# EUROPEAN PATENT APPLICATION

(11) **EP 3 928 782 A1**
(43) Date of publication of application: **29.12.2021**
(21) Application number: 21163701.2
(22) Date of filing: 19.03.2021
(51) Int. Cl.: A61K 35/76, A61P 31/04, C12N 7/00

(54) **BACTERIOPHAGE BASED THERAPY**

(30) Priority: 20.03.2020 EP 20164662
(71) Applicant: Phagegroup LLC, 0159 Tbilisi (GE)
(72) Inventor: Tavartkiladze, Prof. Alexandre, 0159 Tbilisi (GE); Mchedlishvili, Valerian, 0159 Tbilisi (GE)
(74) Representative: Rommeswinkel, Marike

(57) **Abstract**

The present invention relates to the medical field of treating bacterial infections. In particular, the present invention relates to a bacteriophage therapy of the treatment of bacterial infections and/or infectious diseases. In this context, the present invention discloses a specific bacteriophage DSM 33404, capable of infecting bacteria and having lytic activity against bacteria, as well as a pharmaceutical composition containing said bacteriophage for the use in the treatment of bacterial infections and/or infectious diseases.

## Description

The present invention relates to the medical field of the treatment of bacterial infections, especially on the basis of bacteriophage therapy.

Particularly, the present invention relates to a bacteriophage which is capable of infecting a variety of bacteria. Furthermore, the present invention relates to a composition containing a therapeutically effective amount of a bacteriophage according to the present invention. The bacteriophage and/or the composition according to the present invention are suitable for the use in the treatment of bacterial infections, and other infections.

A bacterial infection is a proliferation or growth of a harmful strain or multiple harmful strains of bacteria on or inside the body and can be caused by various pathogenic species of bacteria. The most common bacteria causing healthcare-associated infections belong to the genera or families of *Acinetobacter, Bacteroides, Burkholderia, Clostridium, Enterobacteriaceae, Enterococcus, Escherichia, Klebsiella, Staphylococcus, Streptococcus, Mycobacterium* and *Pseudomonas.* Bacterial infections can occur in any area of the body and are linked with various clinical pictures or symptoms and lead to different illnesses. Common clinical pictures or illnesses caused by bacteria are infections of the respiratory tract (for example pneumonia or bronchitis), wound infections, septicemia, meningitis, ear, nose and throat infections (for example otitis media or tonsillitis), infections of bones and joints (for example arthritis, osteomyelitis or osteitis). Even though bacterial infections regularly hit healthy persons, patients with a weakened immune system, for example oncological patients, diabetes patients or patients with immune deficiencies, are particularly prone for long lasting and tricky to treat bacterial infections, for example in the form of fistulas, furunculosis or wound infections.

According to established therapeutically concepts, bacterial infections are usually treated with antibiotics. An antibiotic is a type of antimicrobial substance which is active against bacteria. The main classes of antibiotics are β-lactam antibiotics, glycopeptide antibiotics, quinolone antibiotics, polyketide antibiotics, aminoglycoside antibiotics, polypeptide antibiotics and/or sulfonamide antibiotics. The antibiotic either kills or inhibits the growth of bacteria, depending on the individual mechanism of action. Generally, the mode of action of antibiotics is rather unspecific, i.e. antibiotics not only target specifically the pathogenic bacteria but also useful bacteria, like the intestinal flora. Due to their low specificity, therapies with antibiotics often go along with several undesired side effects, especially diarrhea and allergic reactions.

Back in the 1940's, the development and application of antibiotics was a milestone in modern medicine, as until then usually fatal infections became easy to treat. Nevertheless, the massive and often unnecessary use of antibiotics in the treatment of illnesses and infectious diseases led to the development and spread of various antibiotic resistances. In the recent years, several bacteria highly resistant to most or all drugs, including the antibiotic of last resort, vancomycin, have been spreading all over the world. These resistances are mainly disseminated by plasmids, transposons and insertion elements. Resistance markers may be transmitted between cells of different species of bacteria.

Particularly, the so-called ESKAPE organisms (*Enterococcus faecium, Staphylococcus aureus, Klebsiella pneumoniae, Acinetobacter baumannii, Pseudomonas aeruginosa* and *Enterobacter spp.*) have become resistant to several antibiotics, especially penicillin, vancomycin and carbapenems. Such resistances, in particular multiple resistances, make the treatment of infections extremely challenging. Thus, antibiotic treatment of infections caused by multi-drug resistant bacteria is often ineffective and the growing resistance of pathogenic bacteria is of great importance in medical practice.

Against this background, there is a need of alternative therapeutic concepts for the treatment of bacterial infections to replace or complement established antibiotic therapy.

One promising alternative attempt to treat bacterial infections is the therapeutic application of bacteriophages. Bacteriophages are viruses that attack or infect bacteria, multiply within the infected bacteria and finally cause disruption of the bacterial cell (lysis). The infection and lytic action of bacteriophages is highly specific. In contrast to antibiotics, bacteriophages are mostly specific for the host organism. Bacteriophage therapy was described for the first time in 1921 by BRUYNOGHE and MAISIN in the treatment of staphylococcal skin infections. Although the results were promising, little was accomplished in this field during the following years. The idea of potential applications of bacteriophage therapy was abandoned after the introduction of sulphonamides and then antibiotics into medical practice.

Against this background, bacteriophage therapy is not well established yet and there exist only a few trial studies with respect to the therapeutic efficacy. Furthermore, many bacteriophages are extremely specific and comprise lytic activity against one or very few strains only. Such high specificity complicates the use of phages in the treatment of bacterial infections in particular with respect to the economic and therapeutic efficiency.

Overall, in consideration of the rapid spread of multi-resistant pathogenic bacteria, there is a strong need for effective therapeutic concepts and/or approaches for the treatment of bacterial infections, in particular tricky to treat and/or chronic bacterial infections with antibiotic resistant bacteria.

Considering this, the problem of the present invention has to be seen in the supply of a new therapeutic concept for the treatment of bacterial infections, particularly infections with antibiotic resistant bacteria.

Especially, the object of the present invention has to be seen in a therapeutic concept for the treatment of bacterial infection, which overcomes the existent problem of effective treatment of infections with antibiotic resistant bacteria and which is further well tolerated and linked with at least reduced side effects in comparison to antibiotics. Furthermore, the new therapeutic concept should be effective against a broad range of bacteria, especially antibiotic resistant bacteria.

The applicant has surprisingly found that the aforementioned problem can be solved on the basis of a pharmaceutical composition as claimed in Claim 1; further, particularly advantageous embodiments of this aspect are subject-matter of the respective depending claims.

Additionally, the present invention relates to a bacteriophage as such according to the respective independent claim; furthermore, in particular advantageous embodiments of this aspect are subject-matter the respective dependent claims.

Furthermore, the present invention relates to a method of treating bacterial infections and/or infection diseases.

With respect to the aspects of the present invention it has to be pointed out that explanations, which have been made in relation to one aspect, self-evidently also apply with respect to the other aspects.

Apart from this, a person skilled in the art can - depending on the application or depending on the individual case - deviate from the specified weights, specified quantities and specified ranges that are stated below without departing from the scope of the present invention.

Moreover, all specified values or specified parameters or the like that are mentioned below can absolutely be ascertained or determined using normed or standardized or explicitly specified determination methods or else using determination or measurement methods familiar per se to a person skilled in the art in this field.

With this being said, the present invention will now be elucidated in detail below.

The applicant surprisingly found out that a Caucasian Origin's BP (synonymously Caucasian Origin's Bacteriophage) as deposited at the DSMZ, Inhoffenstr. 7B, 38124 Braunschweig, Germany, under the accession number DSM 33404 has a multifunctional activity against different species of bacteria and is suitable for the use in a highly efficient treatment of bacterial infections and/or infectious diseases caused by various bacteria species.

Considering this, subject-matter of the present invention is - according to the **first** aspect of the present invention - a bacteriophage for the use in the treatment of bacterial infections and/or infectious diseases, wherein the bacteriophage is a Caucasian Origin's Bacteriophage as deposited at Deutsche Stammsammlung von Mikroorganismen und Zellkulturen GmbH (DSMZ) under the accession no. DSM 33404.

The Caucasian Origin's Bacteriophage according to the present invention has a relatively broad efficacy spectrum against particularly for humans pathogenic bacteria and is therefore suitable for the use in the treatment of various bacterial infections on the basis of a bacteriophage therapy. Nevertheless, unlike antibiotics, the bacteriophage does not infect and lyse beneficial bacteria, for example the intestinal flora or the natural skin flora.

The Caucasian Origin's Bacteriophage according to the present invention belongs to the order of *Ligamenvirales* and the family of *Rudiviridae.* The phage has been isolated from the waters of the mountainous region of western Georgia. The virus is stick or rod-shaped and has a size of about 23 nm in width and 870 nm in length. The genome is composed of linear dsDNA. The unique capsid protein of the Caucasian Origin's Bacteriophage has an amino acid sequence according to SEQ. ID NO. 3. The mRNA sequence coding for the capsid protein is contained in SEQ. ID NO. 4. Additionally, the bacteriophage expresses mRNA sequences according to SEQ. ID NO. 1 and SEQ. ID NO. 2. The Caucasian Origin's Bacteriophage has polyvalent properties. The host spectrum of the Caucasian Origin's Bacteriophage includes at least the following bacterial strains: *Klebsiella pneumoniae, Pseudomonas aeruginosa, Escherichia coli, Enterobacter spp., Acinetobacter baumannii, Salmonella enterica, Proteus vulgaris, Staphylococcus aureus* and *Streptococcus pyogenes.*

As delineated in the following, the phage has due to its polyvalent properties a large host spectrum and can infect and lyse a variety of bacterial strains. The broad host spectrum makes the Caucasian Origin's Bacteriophage and/or lysates of Caucasian Origin's Bacteriophage suitable for the treatment of a variety of bacterial infections and/or infectious diseases, particularly in humans. Especially, the bacteriophage and/or the respective lysates can be used as pharmaceutically active component in compositions for the treatment of bacterial infections and/or infectious diseases. With respect to the host range of the bacteriophage, reference is particularly made to the experimental section of this patent application, especially the in vitro studies.

The bacteriophage according to the present invention, in particular in connection with its use in the treatment of bacterial infections, is linked with several advantages, which are delineated hereinafter:
Even though most bacteriophages are generally highly specific with respect to their lytic action and host strains, the bacteriophage according to the present invention is characterized by multifunctional or polyvalent activity against different species of bacteria. Particularly, the bacteriophage according to the present invention infects several bacteria strains causing healthcare-associated infections and has lytic activity against *Acinetobacter baumannii* (carbapenem-resistant), *Enterococcus faecium, Pseudomonas aeruginosa* (carbapenem-resistant), *Enterobacter spp.* (carbapenem-resistant), *Proteus faecium, Proteus vulgaris, Proteus mirabilis, Salmonella typhimurium, Salmonella enterica, Staphylococcus aureus* (methicillin resistant, vancomycin-intermediate, vancomycin-resistant), *Klebsiella pneumoniae, Streptococcus pyogenes* and *Escherichia coli.* Considering this, the bacteriophage according to the present invention is - in particular when compared to the unspecific mode of action of antibiotics - suitable for a pathogen-specific therapy for bacterial infections caused by the most common bacteria causing healthcare-associated infections.

The specificity of the bacteriophage according to the present invention is particularly advantageous as antibiotic resistances are widely spread in the aforementioned group of bacteria. Particularly, the aforementioned bacteria are often resistant to carbapenem, methicillin, vancomycin and fluoroquinolone. Considering this, the bacteriophage according to the present invention can be used as a potent replacement for or supplement to established antibiotic therapy for the treatment of several infectious diseases caused by the aforementioned bacteria, for example furunculosis, osteomyelitis, infections of connective tissue and lymphatic vessels, fistulas, meningitis, septicemia, otitis media, peritonitis, pyogenic arthritis and myositis, osteitis, pyogenic infections of burns, wound infections and mastitis.

Furthermore, the use of the bacteriophage according to the present invention in the treatment of bacterial infections is linked with only a low risk of undesired side effects. Particularly, the application of the bacteriophage according to the present invention in the treatment of bacterial infections does not lead to the typical side effects of antibiotics, for example allergic reactions or gastrointestinal complaints as a result of the unspecific effect mechanism.

With respect to the therapeutic application of the bacteriophage according to the present invention, a convenient administration of a drug containing the bacteriophage is possible, in particular an intravenous application. Furthermore, also an application per os is suitable as well as a topical application, for example in the treatment of bladder infections.

Furthermore, the Caucasian Origin's Bacteriophage has the ability to widely spread through the body when applied systemically, preferably intravenously. In contrast to most antibiotics, the phage is able to pass through the blood-brain barrier and is therefore a potent medical substance for the treatment of infections of the brain, particularly meningitis or encephalitis. Additionally, the phages according to the present invention are not only superior with respect to their distribution in the body but also with respect to the kinetics. For, unlike antibiotics, bacteriophages are self-amplifying or self-replicating in the presence of the host, i.e. the pathogenic bacteria to be treated.

Overall, the present invention provides a highly efficient alternative to common antibiotic therapy for the treatment of bacterial infections. Particularly, the use of the bacteriophage in the treatment of bacterial infection addresses the problem of the increasing number of antibiotic resistant bacteria.

With respect to the high efficiency of the bacteriophages according to the present invention, reference can also be made to the exemplary embodiments of the present invention, as delineated in the following.

Advantageous and/or preferred embodiments of the present invention will be described in the following. Furthermore, the following terms or definitions are provided to add in the understanding of the present invention.

The term "bacteriophage", synonymously also called phage or bacterial virus, is understood according to the present invention as a virus that infects and replicates within bacteria. Bacteriophages are composed of proteins that encapsulate a DNA or an RNA genome. The bacteriophage according to the present invention has a linear dsDNA genome. Phages usually replicate within the bacterium following the injections of their genome into its cytoplasm. After infection of the bacterial host cell, bacteriophages enter either a lysogenic or lytic lifecycle.

With respect to the present invention, the lytic lifecycle is of particular relevance. Thereby, the phage attaches to the host cell and injects the phage genome. After infection, the phage DNA or RNA circularizes and enters either the lytic or lysogenic cycle. In the case of the lytic cycle, the host cell's DNA is degraded and the cell's metabolism is directed to initiate phage bio synthesis. On the basis of biosynthesis, the phage DNA or RNA is replicated inside the cell, which leads to the expression of new phage DNA and RNA as well as phage proteins. During subsequent maturation, the replicated material assembles to new bacteriophages. Through the final lysis, the newly formed and infectious phages are released from the infected cell. The new phages are capable of infecting further host cells. Through the lysis of the infected cell, the host cell is destroyed and inactivated. For the preparation of a phage lysate for pharmaceutical use and/or a phage stock, the crude lysate is purified and/or filtered to remove cell parts of the lysed bacteria and further undesired bacterial substances, for example toxins. Optionally, sterile filtration and/or a concentration of the phage solution can be performed.

The therapeutic concept of the present invention, i.e. the bacteriophage-based therapy of bacterial infection, is - without being bound to this theory - based on the lytic activity of the phages. In other words, the pathogenic bacteria will be destroyed and inactivated through the phage-initiated cell lysis. With respect to the lytic activity of the Caucasian Origin's Bacteriophage, reference is made to the experimental section of this application.

The bacteriophage of the present invention has a large spectrum of host organisms, particularly bacterial strains belonging to the genera *Staphylococcus, Escherichia, Pseudomonas, Klebsiella, Acinetobacter, Proteus, Enterobacter, Enterococcus, Streptococcus* and/or *Salmonella.* Particularly, the bacteriophage is capable of producing a lytic infection in bacteria of the genera *Staphylococcus, Escherichia, Enterococcus, Pseudomonas, Klebsiella, Acinetobacter, Proteus, Enterobacter, Streptococcus* and/or *Salmonella.*

The term *"Staphylococcus"* in the sense of the present invention means a genus of Gram-positive bacteria. The genera *Staphylococcus* includes about 40 species, wherein most of them are harmless for humans and which reside normally on the skin and the mucus membranes of humans and other organisms. Nevertheless, there are some species of *Staphylococcus* which can lead to severe and even life-threatening bacterial infections.

In this context, the species *Staphylococcus aureus* is of particular relevance. *Staphylococcus aureus* is frequently found in the upper respiratory tract and on the skin. Usually, this species acts as a commensal of the human microbiota without leading to any symptoms. Nevertheless, in particular for people with a weakened immune system or immunocompromised patients, *Staphylococcus* can become a pathogen, leading to severe skin infections, respiratory infections and food poisoning. In the worst case, infections with *Staphylococcus aureus* can lead to life-threatening diseases, such as pneumonia, meningitis, osteomyelitis, endocarditis, toxic shock syndrome, bacteremia and sepsis. Furthermore, many strains of *Staphylococcus aureus* have become resistant to several antibiotics, for example beta-lactam antibiotics, like penicillin or methicillin, or even reserve antibiotics, like vancomycin. As delineated before, the bacteriophage according to the present invention is capable of infecting and/or lysing, particularly bacteria of the species *Staphylococcus aureus.* This applies to cells which are sensitive to antibiotics as well as to antibiotic-resistant strains.

The term *"Escherichia"* means in the sense of the present invention a genus of Gram-negative rod-shaped bacteria from the family *Enterobacteriaceae.* Many species of the genus *Escherichia* are inhabitants of the gastrointestinal tract of animals, in particular also of humans. Even though many *Escherichia* species are harmless for humans, there exists still a number of pathogenic species. Particularly, several strains of the species *Escherichia coli* can be harmful to humans. This applies particularly to humans with a deficient or weakened immune system and/or chronic diseases. Pathogenic varieties of *Escherichia coli* can lead for example to food poisoning, septic shock, meningitis or urinary tract infections. Furthermore, also strains of *Escherichia coli* have become resistant against several antibiotics. The bacteriophage according to the present invention is capable of infecting bacteria of the genus *Escherichia,* in particular *Escherichia coli,* irrespective of the presence of antibiotic resistances of the cells.

The term *"Pseudomonas"* means in the sense of the present invention a genus belonging to the class of *Gammaproteobacteria.* Most of the *Pseudomonas* species are harmless for humans. Nevertheless, the species *Pseudomonas aeruginosa* is an opportunistic human pathogen. *Pseudomonas aeruginosa* can for example infect the airway, the urinary tract, burns and wounds and can cause blood infections. With respect to the clinical picture, infections with *Pseudomonas aeruginosa* can lead to pneumonia, septic shock, urinary tract infections, gastrointestinal infections as well as skin and soft-tissue infections. Particularly vulnerable for infections with *Pseudomonas aeruginosa* are patients with a weakened immune system, elderly people and people with chronic diseases. Additionally, an increasing number of antibiotic resistant strains of *Pseudomonas aeruginosa* makes the treatment of infections difficult.

The term *"Klebsiella"* is understood as a genus of rod-shaped bacteria with a polysaccharide-based capsule. Bacteria of the genus *Klebsiella* relate to the class of *Gammaproteobacteria.* With respect to humans, species of *Klebsiella* often build part of the normal flora of the human nose, mouth and gastrointestinal tract. Nevertheless, some species of *Klebsiella* can behave as opportunistic human pathogens and lead to several diseases, in particular pneumonia, urinary tract infections, sepsis, meningitis, diarrhea, peritonitis and soft-tissue infections. Most of the infections with *Klebsiella* species, which require immediate treatment, are caused by the species *Klebsiella pneunomiae.* Many species of *Klebsiella,* in particular *Klebsiella pneumoniae,* have become resistant to a variety of antibiotics, which further complicates the treatment of infections with *Klebsiella* species, in particular infections with *Klebsiella pneumoniae.*

The term *"Acinetobacter"* in context with the present invention means a genus of Gram-negative bacteria, which also belong to the class of *Gammaproteobacteria.* Usually, species of *Acinetobacter* are harmless for young or middle-aged and healthy humans. Nevertheless, *Acinetobacter* species can behave pathogenic in particular in patients with a weakened immune system and are a key source of infections of debilitated patients in hospitals. In this context, the species *Acinetobacter baumannii* is of particular relevance. Infections with *Acinetobacter baumannii* can lead to different diseases with a variety of symptoms, for example pneumonia, blood infections, meningitis, wound infections and urinary tract infections. Also, species of *Acinetobacter,* in particular *Acinetobacter baumannii,* have become resistant to several antibiotics, in particular against carbapenem. The bacteriophage according to the present invention is capable to infect and lyse cells of *Acinetobacter baumannii* and *Acinetobacter baumannii* carbapenem resistant.

The term *"Proteus"* in connection with the present invention describes a genus of Gram-negative *Proteobacteria. Proteus* species are widely distributed and can be found for example in the human intestine as well as human feces. Most of the *Proteus* species are harmless for healthy humans. However, in patients with a weakened immune system or elderly people species of *Proteus* can behave pathogenic and lead for example to blood infections and infections of the urinary tract. Particularly, the species *Proteus vulgaris* and *Proteus mirabilis* are opportunistic human pathogens, which particularly lead to urinary tract infections and blood infections. The bacteriophage according to the present invention is capable of infecting and/or lysing *Proteus* species, in particular *Proteus vulgaris* as well as *Proteus mirabilis.*

The term *"Enterobacter"* relates in connection with the present invention to a genus of Gram-negative bacteria of the family *Enterobacteriaceae.* Many strains of *Enterobacter* species are pathogenic and cause opportunistic infections in humans with a weakened immune system or chronic diseases. Species of *Enterobacter* often infect the urinary and respiratory tract. Furthermore, the development of resistances against several antibiotics makes the treatment of patients with infections with *Enterobacter* species complicated. Many strains of *Enterobacter* species have become resistant to carbapenem, which is usually the antibiotic of choice for the treatment of *Enterobacter* infections. The bacteriophage of the present invention is capable of infecting and/or lysing species of *Enterobacter,* in particular carbapenem-resistant *Enterobacter* species.

The term *"Streptococcus"* in the sense of the present invention means a genus of Gram-positive spherical bacteria. Species belonging to the genus of *Streptococcus* can lead to a variety of bacterial infections and/or infectious diseases particularly in humans. Some species of *Streptococcus* can even have hemolytic properties. Medically relevant *Streptococci* particularly include *Streptococcus pyogenes,* which causes pharyngitis, cellulitis and erysipelas, *Streptococcus mitis,* which causes endocarditis, *Streptococcus mutans,* which causes dental caries, and *Streptococcus pneumoniae,* which causes pneumonia. The bacteriophage of the present invention is capable of infecting bacteria of the genus *Streptococcus,* particularly *Streptococcus pyogenes.*

The term *"Salmonella"* in the sense of the present invention means a genus of rod-shaped Gram-negative bacteria belonging to the family *Enterobacteriaceae. Salmonella* species are facultative intracellular pathogens and can invade several cell types, for example epithelial cells, macrophages, dendritic cells and M cells. Most of the infections with *Salmonella* are caused by strains of the species *Salmonella enterica.* The most common path of infection is food, which is infected with *Salmonella enterica,* particularly cattle and poultry as well as raw eggs. Infections with *Salmonella* species, in particular *Salmonella enterica* or *Salmonella typhimurium* (syn: *Salmonella enterica spp. enterica* ser. *typhimurium)* lead to an inflammation of the gastrointestinal tract caused by the toxins produced by the bacteria. Infections with species of the genus *Salmonella* do not only occur in weakened or elderly patients or humans, but also in children or healthy adults, thereby leading to severe symptoms. The bacteriophage of the present invention is capable of infecting bacteria of the genus *Salmonella,* particularly *Salmonella enterica* and/or *Salmonella typhimurium.*

Overall, as can be seen from the above, the bacteriophage of the present invention has a broad host spectrum and is therefore highly efficient in the treatment of infections with the above described bacteria. Particularly, the bacteriophage is capable to produce lytic infection in several so-called ESKAPE organisms. ESKAPE organisms often lead in weakened and hospitalized patients with chronic diseases to severe to life-threatening infections. ESKAPE organisms are commonly associated with antimicrobial resistances, particularly against penicillin, methicillin, vancomycin and carbapenems. Considering the specificity of the bacteriophage according to the present invention, the phage is linked with a high pharmaceutical efficacy in the treatment of such infections as the phage also infects antibiotic resistant bacteria of the aforementioned genera in species. This is quite surprising as most of the phages have a limited specificity, which is particularly limited to a single strain.

In connection with the host spectrum of Caucasian Origin's Bacteriophage, reference is made to the experimental section. The studies performed by the applicant show that the phage has lytic activity and/or antimicrobial activity at least against *Escherichia coli, Salmonella enterica, Streptococcus pyogenes, Klebsiella pneumoniae, Proteus vulgaris, Pseudomonas aeruginosa, Acinetobacter baumannii* and *Staphylococcus aureus.*

Having this said, particularly advantageous embodiments of the present invention and characteristics of the bacteriophage are delineated in the following.

According to a preferred embodiment of the present invention, the bacteriophage is capable of infecting, especially capable of producing a lytic infection in bacteria with antibiotic resistances, in particular single or multiple antibiotic resistances. Particularly, the bacteriophage according to a further preferred embodiment of the present invention is capable of infecting, especially capable of producing a lytic infection in bacteria with resistances against β-lactam antibiotics, glycopeptide antibiotics, quinolone antibiotics, polyketide antibiotics, aminoglycoside antibiotics, polypeptide antibiotics and/or sulfonamide antibiotics, in particular methicillin, vancomycin, carbapenem and/or fluoroquinolone.

According to a particularly preferred embodiment of the present invention, the bacteriophage is capable of infecting, especially capable of producing a lytic infection in *Klebsiella pneumoniae, Pseudomonas aeruginosa* carbapenem-resistant, *Pseudomonas aeruginosa, Escherichia coli, Enterobacter spp., Enterobacteriaceae* carbapenem-resistant, *Enterococcus faecium, Acinetobacter baumannii* carbapenem-resistant, *Salmonella typhimurium, Salmonella enterica, Proteus vulgaris, Proteus mirabilis, Staphylococcus aureus, Staphylococcus aureus* methicillin-resistant, *Staphylococcus aureus* vancomycin-intermediate, *Staphylococcus aureus* vancomycin-resistant and/or *Streptococcus pyogenes.*

According to another preferred embodiment of the present invention, the bacteriophage expresses a nucleic acid sequence, particularly an mRNA sequence, having a nucleotide sequence which has at least 70 %, in particular at least 80 %, preferably at least 85 %, more preferred at least 90 %, further preferred at least 95 %, mostly preferred at least 99 % identity to SEQ. ID. NO. 1. Likewise, the bacteriophage can express a nucleic acid sequence, particularly an mRNA sequence, having a nucleotide sequence which has at least 70 %, in particular at least 80 %, preferably at least 85 %, more preferred at least 90 %, further preferred at least 95 %, mostly preferred at least 99 % identity to SEQ. ID. NO. 2.

According to a particularly preferred embodiment of the present invention, the bacteriophage expresses a nucleic acid sequence, particularly an mRNA sequence, having a nucleotide sequence which has at least 70 %, in particular at least 80 %, preferably at least 85 %, more preferred at least 90 %, further preferred at least 95 %, mostly preferred at least 99 % identity to SEQ. ID. NO. 1 and SEQ. ID. NO. 2.

Overall, it is apparent that the bacteriophage according to the present invention is suitable for the use in the treatment of bacterial infections, in particular bacterial infection caused by bacteria of the genera *Staphylococcus, Enterobacter spp., Enterococcus, Escherichia, Pseudomonas, Klebsiella, Acinetobacter, Proteus, Streptococcus* and/or *Salmonella.* Particularly, the bacteriophage can be used in the treatment of infections caused by *Klebsiella pneumoniae, Pseudomonas aeruginosa* carbapenem-resistant, *Pseudomonas aeruginosa, Escherichia coli, Enterobacter spp., Enterobacteriaceae* carbapenem-resistant, *Enterococcus faecium, Acinetobacter baumannii* carbapenem-resistant, *Salmonella typhimurium, Salmonella enterica, Proteus vulgaris, Proteus mirabilis, Staphylococcus aureus, Staphylococcus aureus* methicillin-resistant, *Staphylococcus aureus* vancomycin-intermediate, *Staphylococcus aureus* vancomycin-resistant and/or *Streptococcus pyogenes.*

Considering the host range of Caucasian Origin's Bacteriophage, the bacteriophage according to the present invention and/or lysates prepared from that phage are suitable for the use in the treatment of various diseases and symptoms caused by bacterial infections, in particular bacterial infections with the above named bacteria. According to a preferred embodiment of the present invention, the bacteriophage is suitable for the use in the treatment of furunculosis, osteomyelitis, infections of connective tissue, infections of lymphatic vessels, fistulas, septicemia, meningitis, otitis, peritonitis, arthritis, myositis, osteomyelitis, osteitis, infections of wounds and/or burns, infections of skin and/or mastitis.

In this context, reference is already made at this point to the experimental section of this patent application. The experimental studies have been performed with the respective Caucasian Origin's BP as deposited at the DSMZ, Inhoffenstr. 7B, 38124 Braunschweig, Germany, under the accession number DSM 33404. The experimental section shows that the phage has a high therapeutic efficacy in the treatment of bacterial infections with *Staphylococcus aureus, Salmonella typhimurium, Salmonella enterica, Klebsiella pneumoniae, Escherichia coli, Proteus mirabilis, Proteus vulgaris, Enterobacter spp., Enterococcus faecium, Pseudomonas aeruginosa, Streptococcus pyogenes* and/or *Acinetobacter baumannii.*

Overall, the present invention provides a highly efficient alternative therapeutic concept for the treatment of bacterial infections. The bacteriophage is suitable for the use in the treatment of bacterial infections caused by several different bacterial strains, due to the broad specificity of the phage. Particularly, the bacteriophage as such and/or a treatment on the basis of the bacteriophage address the problem of the increasing number of antibiotic resistant bacteria.

Furthermore, the present invention relates - according to a **second** aspect of the present invention - to a pharmaceutical composition for the use in the treatment of bacterial infections and/or infectious diseases, wherein the composition contains a therapeutically effective amount of a bacteriophage, wherein the bacteriophage is a Caucasian Origin's Bacteriophage as deposited at Deutsche Stammsammlung von Mikroorganismen und Zellkulturen GmbH (DSMZ) under the accession no. DSM 33404.

In other words, the present invention relates to a pharmaceutical composition, wherein the composition contains the bacteriophage according to the present invention as described above as the pharmaceutically active component.

Particularly, the pharmaceutical composition contains a therapeutically effective amount of Caucasian Origin's Bacteriophage, wherein the amount is sufficient to infect bacteria of the genera *Staphylococcus, Escherichia, Pseudomonas, Klebsiella, Acinetobacter, Proteus, Enterobacter, Streptococcus* and *Salmonella.*

The amount of active or infectious bacteriophages in the compositions according to the present invention can vary in wide ranges. Particularly good results in the treatment of bacterial infections are achieved when the composition of the present invention has a phage titer in the range of 1 to 10¹⁵ PFU/ml, especially 10² to 10¹³ PFU/ml, in particular 10³ to 10¹¹ PFU/ml, preferably 10⁴ to 10⁹ PFU/ml, particularly preferred 10⁵ to 10⁷ PFU/ml, based on the composition. The aforementioned phage titer are therapeutically effective amounts in the sense of the present invention.

The term "plaque-forming unit (PFU)" is understood according to the present invention as a measure for the number of particles, i.e. bacteriophages, capable of forming plaques per unit volume. The number of plaques in a nutrient medium is used for virus quantification and therefore an indicator for the number of infectious virus particles in a defined volume. A viral plaque is in this context a visible structure formed within the cell culture, for example bacterial cultures within some nutrient medium. After infecting and penetrating the bacteria, the bacteriophages replicate and spread. Thereby, regions of cell deconstruction are generated. These regions are called plaques. To determine the virus titer, the plaques are counted. On the basis of the plaque number, the number of infectious phages in a defined volume of a phage stock or composition is determined. In this context, for example, a PFU of 10³ means that one milliliter of the composition contains 10³ bacteriophages, which are capable of infecting bacteria. Additionally, it is indicated that "PFU" is an established term in microbiology and virology. Therefore, no further explanations are necessary for the skilled practitioner with respect to this term.

Preferably, the composition comprises the Caucasian Origin's Bacteriophage in the form of a phage lysate. In this context, it is preferred when the lysate has been obtained from Caucasian Origin's Bacteriophage infected *Staphylococcus aureus, Pseudomonas aeruginosa, Proteus vulgaris, Escherichia coli, Acinetobacter baumannii, Klebsiella pneumoniae, Enterobacter* spp. and/or *Staphylococcus pyogenes.*

According to a particularly preferred embodiment, the composition according to the present invention comprises the bacteriophage on the basis of a combination of lysates, wherein the lysates have been obtained from Caucasian Origin's Bacteriophage infected *Staphylococcus aureus, Pseudomonas aeruginosa, Proteus vulgaris, Escherichia coli, Acinetobacter baumannii, Klebsiella pneumoniae, Enterobacter* spp. and *Staphylococcus pyogenes.*

Furthermore, it is preferred when the composition contains the Caucasian Origin's Bacteriophage as the only pharmaceutically active ingredient. Particularly, it is preferred when the composition does not comprise further species of bacteriophages. According to another, alternative embodiment, the composition can comprise further pharmaceutically active substances, preferably substances with antimicrobial properties. Such further antimicrobial components can be selected from antibiotics, antiseptics, antimycotics and the like.

With respect to the further characteristics of the composition according to the present invention, it is preferred when the composition is present as an aqueous composition and/or when the composition is aqueously based and/or is present as an aqueous formulation, especially in the form of an aqueous solution. Likewise, it is preferred when the composition contains water, especially purified water, and/or that the composition contains water as pharmaceutically compatible excipient. Studies performed by applicant have shown that the bacteriophage according to the present invention is particularly stable when diluted in aqueous compositions. Additionally, it is preferred when the composition according to the present invention contains amino acids.

The amount of water in the composition according to the present invention can vary within wide ranges. According to the invention, it is preferred when the composition has a content of water, especially purified water, of at least 60 % by volume, especially at least 70 % by volume, preferably at least 75 % by volume, particularly preferably at least 80 % by volume, even more preferred at least 88 % by volume, based on the composition.

In order to improve the compatibility and tolerability of the compositions according to the present invention when applied to the patients, it is preferred when the composition is present as an isotonic composition, especially solution, in relation to preferably mammalian, in particular human blood and/or blood cells.

In other words, according to an advantageous embodiment, the composition according to the present invention has an osmotic pressure which is identical to the osmotic pressure of mammalian, in particular human blood and or blood cells.

In order to maintain the isotonicity and/or osmotic pressure of the composition according to the present invention, it is preferred when the composition contains pharmaceutically acceptable, osmotically active substances. In this context, the composition preferably comprises at least one physiologically safe or acceptable, osmotically active substance and/or at least one physiologically safe or acceptable osmolyte. According to a further preferred embodiment, the osmotically active substance and/or osmolyte is selected from the group of ions of physiologically safe or acceptable salts, preferably alkali ions, in particular sodium ions and chloride ions; sugars, in particular glucose; polyols, in particular glycerol; and/or amino acids. Specifically, good results are achieved, when the composition according to the present invention contains a combination of sodium chloride, glucose and amino acids as osmotically active substances.

Concerning the amount of osmotically active substances in the composition, this can vary within wide ranges. According to the invention, it is preferred when the composition according to the invention contains mono- and/or disaccharides, preferably glucose, in amounts in the range of 0.1 to 25 % by weight, especially 0.5 to 20 % by weight, preferably 1 to 15 % by weight, by preference 2 to 10 % by weight, particularly preferably 3 to 8 % by weight, based on the composition.

Likewise, it is preferred when the composition according to the invention contains ions of physiologically safe or acceptable salt, in particular sodium ions and chloride ions, in amounts in the range of 0.001 to 5 % by weight, especially 0.01 to 2.5 % by weight, preferably 0.05 to 1.5 % by weight, particularly preferred 0.1 to 1 % by weight, based on the composition.

Additionally, it is preferred when the composition comprises an amino acid solution. Particularly good results are achieved, when the composition comprises an amino acid solution in amounts in the range of 0.01 to 30 % by volume, especially 0.1 to 25 % by volume, preferably 1 to 20 % by volume, preferred 5 to 15 % by volume, particularly preferred 8 to 12 % by volume, based on the composition.

Furthermore, according to another preferred embodiment of the composition according to the present invention, the composition comprises at least one customary pharmaceutical additive and/or excipient, which is preferably selected from the group of processing aids, stabilizers, emulsifiers, antioxidants, preservatives, humectants, pH setters, pH buffer substances, thickeners, antiseptics, dyes, buffer substances, odorants, fragrances, extenders, binders, wetting substances and/or preservatives and the combinations thereof.

In particular, it is preferred when the composition comprises a preservative, preferably in a concentration in the range of from 0.0001 to 2 % by weight, especially 0.001 to 1 % by weight, preferably 0.005 to 0.1 % by weight, particularly preferably 0.01 to 0.05 % by weight, based on the composition. A suitable preservative is for example quinosole.

Furthermore, with respect to the application of the pharmaceutical composition of the present invention as such, all kinds of established application forms are possible. According to a preferred embodiment of the present invention, the composition is prepared for an intravenous, subcutaneous, intramuscular, oral, parenteral and/or topical application, especially an intravenous, topical and/or oral application, preferably an intravenous and/or oral application.

Overall, the composition according to the present invention can be prepared for any pharmaceutical dosage form. Considering this, the composition can be customized for an optimized dosage form for the respective infection to be treated.

As delineated before, the bacteriophage according to the present invention has a broad specificity of host cells. Against this background, also the compositions according to the present invention containing the respective bacteriophage are suitable for the use in the treatment of several different bacterial infections. Particularly, the pharmaceutical compositions are suitable for the treatment of infections caused by bacteria of the genera *Staphylococcus, Enterobacter, Enterococcus, Escherichia, Pseudomonas, Klebsiella, Acinetobacter, Proteus, Streptococcus* and/or *Salmonella.* According to a further preferred embodiment, the compositions according to the present invention are suitable for the use in the treatment of bacterial infections caused by bacteria selected from *Klebsiella pneumoniae, Pseudomonas aeruginosa* carbapenem-resistant, *Pseudomonas aeruginosa, Escherichia coli, Enterobacter spp., Enterobacteriaceae* carbapenem-resistant, *Enterococcus faecium, Acinetobacter baumannii* carbapenem-resistant, *Salmonella typhimurium, Salmonella enterica, Proteus vulgaris, Proteus mirabilis, Staphylococcus aureus, Staphylococcus aureus* methicillin-resistant, *Staphylococcus aureus* vancomycin-intermediate, *Staphylococcus aureus* vancomycin-resistant and/or *Streptococcus pyogenes.*

Considering that the composition is pharmaceutically active against the abovemen-tioned bacteria, the composition can be used for the treatment of several infectious diseases. According to a preferred embodiment of the present invention, the pharmaceutical composition according to the present invention is used in the treatment of furunculosis, osteomyelitis, infections of connective tissue, infections of lymphatic vessels, fistulas, septicemia, meningitis, otitis, peritonitis, arthritis, myositis, osteomyelitis, osteitis, infections of wounds and/or burns, infections of skin and/or mastitis.

For further details concerning this aspect of the invention, reference can be made to the above explanations in relation to the aspect outlined before, said explanations also applying accordingly with regard to this aspect of the present invention.

Furthermore, the present invention relates - according to a **third** aspect - to a method of treating bacterial infections, comprising administering to a subject in need thereof a Caucasian Origin's Bacteriophage as deposited at Deutsche Stammsammlung von Mikroorganismen und Zellkulturen GmbH (DSMZ) and as described above and/or a composition according to the present invention as described above.

In this context, the bacterial infections are particularly caused by bacteria of the genera *Staphylococcus, Enterobacter, Enterococcus, Escherichia, Pseudomonas, Klebsiella, Acinetobacter, Proteus, Streptococcus* and/or *Salmonella,* preferably caused by *Klebsiella pneumoniae, Pseudomonas aeruginosa* carbapenem-resistant, *Pseudomonas aeruginosa, Escherichia coli, Enterobacter spp., Enterococcus faecium, Enterobacteriaceae* carbapenem-resistant, *Acinetobacter baumannii* carbapenem-resistant, *Salmonella typhimurium, Salmonella enterica, Proteus vulgaris, Proteus mirabilis, Staphylococcus aureus, Staphylococcus aureus* methicillin-resistant, *Staphylococcus aureus* vancomycin-intermediate, *Staphylococcus aureus* vancomycin-resistant and/or *Streptococcus pyogenes.*

Considering the large host spectrum of the bacteriophage, a variety of diseases caused by bacterial infections can be treated on the basis of the method of the present invention. Particularly, the method can be used in the treatment of furunculosis, osteomyelitis, infections of connective tissue, infections of lymphatic vessels, fistulas, septicemia, meningitis, otitis, peritonitis, arthritis, myositis, osteomyelitis, osteitis, infections of wounds and/or burns, infections of skin and/or mastitis.

For further details concerning this aspect of the invention, reference can be made to the above explanations in relation to the aspects outlined before, said explanations also applying accordingly with regard to this aspect of the present invention.

The following working examples better illustrate the subject-matter of the present invention, and they should not be considered limiting the application.

### Experimental Section:

On the basis of several in vitro studies, the lytic and antimicrobial activity of the Caucasian Origin's Bacteriophage have been analyzed. Furthermore, the bacteriophage according to the present invention has been used for the preparation of pharmaceutical compositions particularly for the use in the treatment of bacterial infections. The compositions have been tested with respect to their efficacy in the treatment of different bacterial infections. For the below delineated in vitro studies as well as the efficacy studies, a Caucasian Origin's BP as deposited at the Deutsche Stammsammlung von Mikroorganismen und Zellkulturen GmbH (DSMZ) under the accession number no. 33404 has been used.

### 1. Cultivation of Bacteriophages

Prior to the preparation of compositions according to the present invention and as preparation of the in vitro studies, the bacteriophage was cultivated and isolated. For the purpose of cultivation of host bacteria and the bacteriophage according to the present invention, the following cultivation media have been used:

**Special Medium (SM):**

| | |
|---|---|
| 5.8 g | NaCl |
| 2.0 g | MgSO4 · 7 H₂O |
| 4.5 ml | 1 M Tris HCI pH 7 |
| 5.0 ml | 2 % gelatin |
| q.s. to 1 liter, sterilize by autoclaving | |

**LEM:**

| | |
|---|---|
| 10.0 g | Bacto-tryptone |
| 5.0 g | Yeast extract |
| 5.0 g | NaCl |
| 10.0 ml | 1 M MgSO₄ |
| q.s. to 1 liter, sterilize by autoclaving | |

**LB-Medium:**

| | |
|---|---|
| 10.0 g | Tryptone |
| 5.0 g | Yeast extract |
| 10.0 g | NaCl |
| q.s. to 1 liter, sterilize by autoclaving | |

Top agarose: 0.7 % agarose in LEM
Bottom agar: 1.5 % agar in LEM, pour into 100 mm petri dishes after autoclaving.

### Bacterial cultures for phage purification

For the preparation of bacterial cultures, an overnight culture of the selected bacteriophage's host cell species (for example *Staphylococcus aureus, Salmonella typhimurium, Salmonella enterica, Klebsiella pneumoniae, Escherichia coli, Proteus mirabilis, Proteus vulgaris, Enterobacter spp., Enterococcus faecium, Pseudomonas aeruginosa, Streptococcus pyogenes* or *Acinetobacter baumannii)* is cultivated as starter culture. The overnight culture is further used for inoculation of either a 3 hour culture or another fresh overnight culture. For the 3 hour culture, a 1 : 10 dilution of the overnight culture is cultivated in LB media for 3 hours at 37 °C.

The fresh overnight culture or the 3 hour culture are used for phage amplification. Phage amplification can be performed by following the general procedure for plating and titering of Lambda stocks. The bacteria on which the strain is to be grown are cultured overnight by sterile transfer from a stab or slant to a medium size culture tube containing 5 ml LB with 0.1 M MgSO₄ and, optionally, further additions, for example 1 µg/ml for thiamine or 5 µg/ml thymine or thymidine. The culture is grown about 14 hours at 37 °C. When bacteria are ready, dilute the phage in SM to a final phage concentration of about 2 · 10³ PFU/ml. Dilutions are done by adding 0.05 ml to 5 ml of SM for a 102 dilution or by adding 0.5 ml to 4.5 ml SM for a tenfold dilution.

0.1 ml of the phage dilution are added to 0.25 ml of the 3 hour culture and preabsorbed at 37 °C for 5 to 10 min. 2.5 ml top agarose (melted and tempered at 45 °C to 47 °C) are added to each tube and poured onto bottom agar plates. If thiamine is a requirement, it is added to the top agar. The plates are incubated at 37°C overnight. If the bacterium is temperature sensitive, the preabsorption and overnight incubation is performed at an appropriate temperature.

The purification of phage infected strains can be performed according to established purification protocols. For purification of a single strain from a heterogeneous stock a single, well isolated plaque is selected. The plaque is removed from the plate using a sterile open-ended capillary tube or pasteur pipette to pierce the agar (both top and bottom layers). The plaque is expelled into 1.0 ml SM plus a drop of chloroform and shaken briefly to suspend the phage. One single plaque usually contains an average of 10⁵ to 10⁶ phages. For the next cycle of purification, a 10³ and 10⁴ dilution of the suspension is plated out. At least two cycles are carried out.

### Single plaque isolation / preparation of small lysate

A single plaque is cored out and added to 1 ml SM. 0.5 ml phage are added to 0.15 ml of a 3 hours culture of appropriate bacteria. Bacteria and phage are incubated for 20 min at 37 °C. 3 ml of top agarose are added. The mixture is plated out on a fresh agar plate. Within approximately 4 hours, bacteria growth is apparent. After 1 to 2 more hours, substantial clearing is apparent. When almost all, but not all, bacteria have been lysed, 3 ml SM are added to the plate. The plate is stored at 4 °C. After 1 hour to overnight, the SM is taken off with a sterile pasteur pipet. The resulting stock of phage has a titer of approximately 5 x 10⁹ PFU/ml.

### Isolation via CsCI gradient / preparation of large Ivsate

For the preparation of a large lysate, an overnight culture of the selected bacteriophage's host cell species is grown overnight. The bacteria, the phage and cultivation medium are preabsorbed at 37 °C for 10 to 20 min. For this purpose, 5 ml bacteria and 2 x 10⁷ phages are inoculated. 500 ml LEM are pre-warmed at 37 °C in a 2 liter flask. The inoculant is added to the media and shaken vigorously at 37 °C until lysis is complete (4 or 5 hours to overnight). After lysis, 4 ml per 500 ml media chloroform and NaCl are added to lyse remaining cells. The mixture is shaken for 15 min. at 37 °C and chilled afterwards. The mixture is centrifuged for 15 min. with 8.000 rpm. After centrifugation, polyethylene glycol (PEG) 6000 is added to 7 % by weight to the supernatant. The PEG is dissolved by shaking. The mixture is incubated at cold temperatures for at least 1 hour. After incubation, the supernatant is removed by centrifugation for 15 min. with 8.000 rpm. All residual fluid is wiped out and the pellet is resuspended in 7 ml SM. The suspended pellet is transferred to a centrifuge tube. Include the clumps. Crude DNase is added with 20 µg/ml. The suspension with DNase is incubated for 20 min. at room temperature. Cell debris is removed through centrifugation for 15 min. with 8.000 rpm. After centrifugation, the pellet is discarded and the supernatant is poured into a new tube. 0.78 g CsCI are added per ml of solution. Afterwards, the mixture is centrifuged for 20 hours with 35.000 rpm. The phage band is taken off and dialyzed against SM. 50 to 100 µl of the phage are saved as a high titer stock for future endeavors. The remaining solution is adjusted to 10 mM excess EDTA, 0.1 % SDS. Proteinase K is added to 100 µg/ml and incubate at 65 °C for 45 min. After incubation, the solution is cooled and extracted with phenol, wherein a phenol : chloroform ratio of 50 : 50 is used. Afterwards, ether extraction with subsequent ethanol precipitation either by spooling out or spinning down the DNA is performed. After a wash step with ethanol, the DNA is taken up and solved in standard DNA buffer.

### 2. In Vitro Studies

On the basis of several in vitro studies, the broad host range and the polyvalent properties of the Caucasian's Origin Bacteriophage (DSMZ accession no. DSM 33404) and/or its activity against several different species have been further analyzed. For the in vitro studies, phage lysates with a phage titer of 1 x 10⁷ PFU/ml have been used.

### Growth Experiments

In order to study the perceptibility of different bacteria species towards the Caucasian Origin's Bacteriophage (accession no. DSM 33404), growth experiments have been performed. For this purpose, the growth of polyresistant cultures of *Staphylococcus aureus, Acinetobacter baumannii* (carbapenem-resistant), *Pseudomonas aeruginosa* (carbapenem-resistant) and *Salmonella enterica* (fluoroquinolone-resistant) on growth media containing the bacteriophage has been analyzed.

For this purpose, agar plates have been prepared as growth media with three different concentrations of the bacteriophage lysate (0.01 wt.-%, 0.16 wt.-% and 0.23 wt.-%, based on the growth media, of a bacteriophage lysate with a phage titer of 1 x 10⁷ PFU/ml phages). Each agar plate contained 10 g of growth media. As a control, agar plates without the addition of bacteriophage have been used. In order to analyze the antibacterial activity of the bacteriophage, fluid cultures of polyresistant cultures of *Staphylococcus aureus, Acinetobacter baumannii* (carbapenem-resistant), *Pseudomonas aeruginosa* (carbapenem-resistant) and *Salmonella enterica* (fluoroquinolone-resistant) have been plated out on the agar plates. The agar plates have been incubated for 3 days at the optimal growth temperature of the respective bacteria. After cultivation, the agar plates have been analyzed with respect to bacterial growth. An inhibition of growth is an indicator for the antimicrobial properties of the bacteriophage. The respective results are contained in **table 1** below:

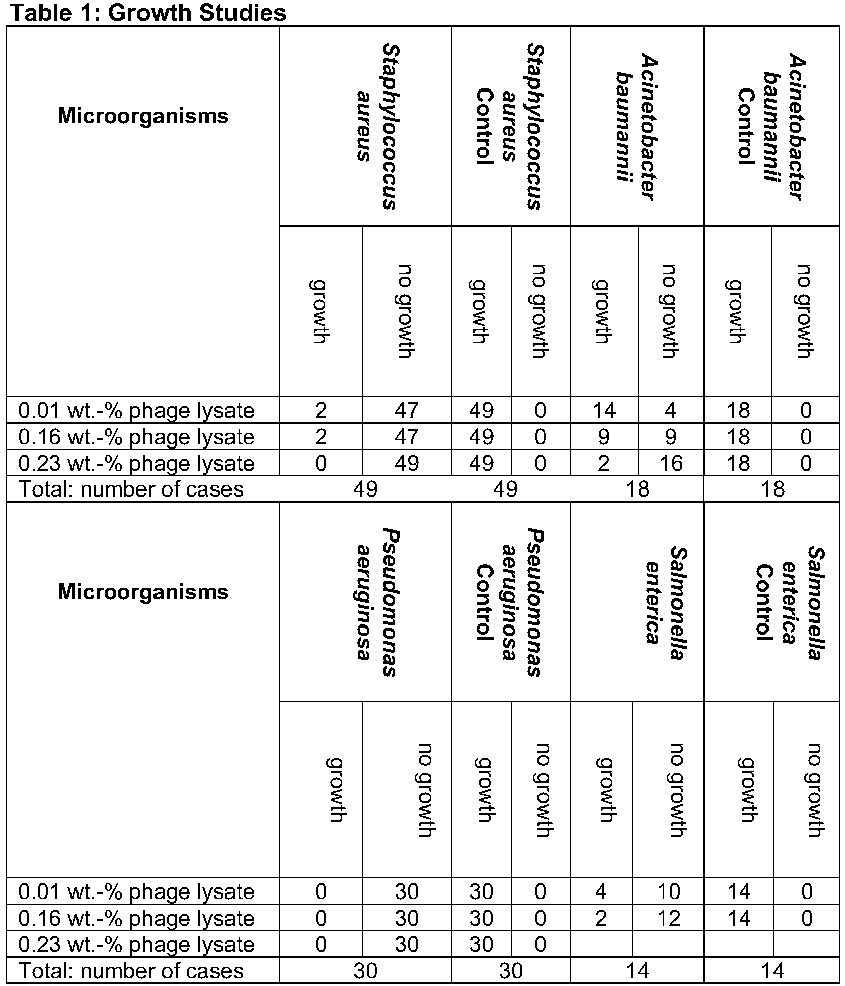

As can be seen from **Table 1** above, Caucasian Origin's Bacteriophage has the ability to inhibit the growth of the tested bacterial strains. It can be summarized that Caucasian Origin's Bacteriophage has lytic properties against a variety of bacteria, in particular *Staphylococcus aureus, Acinetobacter baumannii, Pseudomonas aeruginosa* and *Salmonella enterica.*

Additionally, the growth of *Staphylococcus aureus, Acinetobacter baumannii, Pseudomonas aeruginosa* and *Salmonella enterica* on growth media containing different kinds of antibiotics, on the one hand, and growth media containing the bacteriophage, on the other hand, have been compared as well. For this purpose, agar plates have been prepared with several antibiotics in three different concentrations (Azlocillin, Piperacillin/Tazo, Sultamicillin, Oxacillin, Flucloxacillin, Amoxicillin, Clavulanic acid, Ceftriaxone, Meropem, Cefepime, Cefotaxime, Cefuroxime, Cefoperazone, Vancomycin, Gentamicin, Amikacin, Azithromycin, Roxithromycin, Rovamycine, Clarithromycin, Moxifloxacin, Ofloxacin, Ciprofloxacin, Doxycycline, Rifampin, Linomycin, Imipenem/Cilastin). Thereby, the three different concentrations of the aforementioned antibiotics were individually adapted to the respective substance. The concentrations for each antibiotic (unit: mg/L) are indicated in the table below. Furthermore, agar plates containing three different concentrations of Caucasian Origin's Bacteriophage have been prepared. The solid growth media (agar plates) contained 0.9 wt.-%, 1.6 wt.-% and 2.3 wt.-% of a phage stock/lysate with a phage titer of 1 x 10⁷ PFU/ml. Bacteria of the aforementioned species have been plated out on the agar plates and incubated for 3 days. As a control, agar plates without antibiotics and without bacteriophage have been used. After 3 days, the growth of the bacteria has been analyzed and compared. The respective results are contained in **Table 2** below. In this context, "R" means resistant (= regular growth), "S" means sensitive or susceptible (= no growth) and "MS" means intermediate sensitivity (= reduced growth).

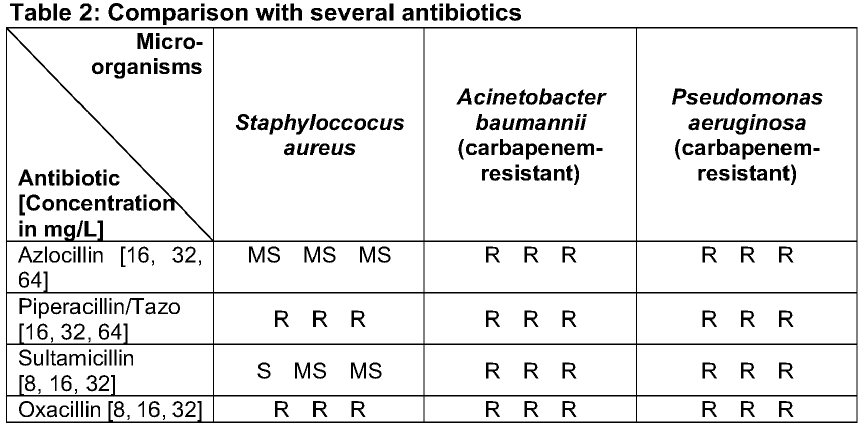

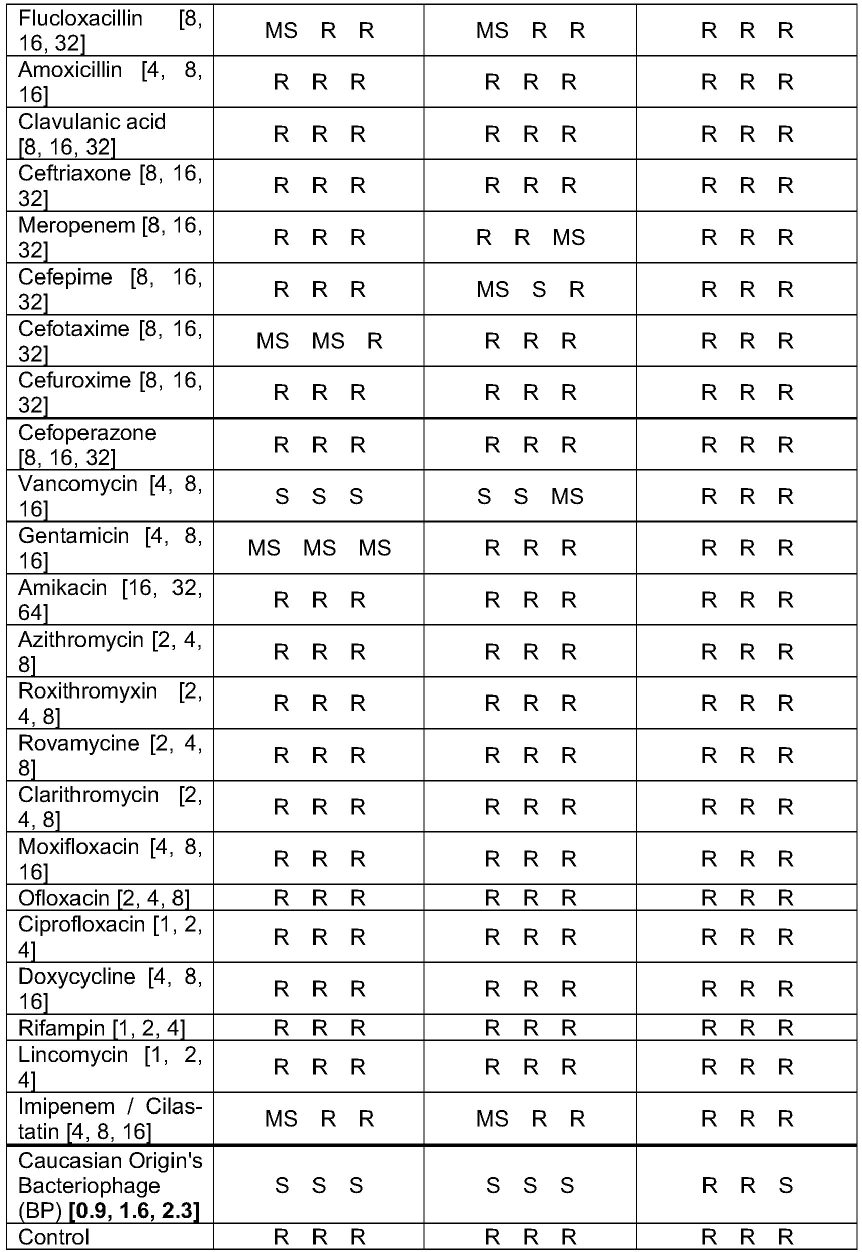

As can be seen from **Table 2,** the tested bacterial strains are resistant to several of the tested antibiotics. In contrast to this, Caucasian Origin's Bacteriophage is capable to inhibit the growth of the tested bacterial strains.

### b) Lysis Studies

Additionally, the capability of Caucasian Origin's Bacteriophage to lyse bacteria of the species *Staphylococcus aureus, Pseudomonas aeruginosa, Proteus vulgaris, Escherichia coli, Acinetobacter baumannii, Klebsiella pneumoniae, Enterobacter* spp. and *Staphylococcus pyogenes* has been analyzed. For this purpose, host bacteria have been isolated from the blood of 50 patients suffering from acute bacterial sepsis. Thereby, 35 patients suffered from mono infections. The remaining 15 patients suffered from mixed infections. 50 isolates of each of the bacteria species mentioned before have been cultivated for lysis studies in 10 ml solid standard media on petri dishes. In order to analyze the capability of the phage to lyse the aforementioned species, a phage fluid lysate with a titer of 1 x 10⁷ PFU/ml has been prepared. 0.2 µl of the bacteriophage solution or lysate have been added to the bacterial cultures. Bacteria cultures and bacteriophage were incubated for 3 days. The number of lysed pathogen isolates were counted. Furthermore, the latent period in minutes was analyzed as well as the productivity of the infection (PFU/cell or phage yield per cell). The respective results are contained in **Table 3** below.

**Table 3: Lysis studies**

| **Number of lysed pathogen isolates** | **Latent period in minutes** | **Productivity [PFU/cell]** | **Host Bacteria** |
|---|---|---|---|
| 48 (50) | 45 | 170 | *Staphylococcus aureus* |
| 44 (50) | 49 | 110 | *Pseudomonas aeruginosa* |
| 42 (50) | 57 | 60 | *Proteus vulgaris* |
| 46 (50) | 60 | 110 | *Escherichia coli* |
| 37 (50) | 55 | 150 | *Acinetobacter baumannii* |
| 43 (50) | 47 | 120 | *Klebsiella pneumoniae* |
| 45 (50) | 35 | 70 | *Enterobacter* spp. |
| 44 (50) | 33 | 47 | *Streptococcus pyogenes* |

It can be seen from **Table 3** that Caucasian Origin's Bacteriophage is highly capable of lysing bacteria belonging to the species *Staphylococcus aureus, Pseudomonas aeruginosa, Proteus vulgaris, Escherichia coli, Acinetobacter baumannii, Klebsiella pneumoniae, Enterobacter* spp. and *Staphylococcus pyogenes.* Additionally, the phage has a relatively short latent period when infecting the aforementioned bacteria. The short latent period is advantageous with respect to the therapeutical use of Caucasian Origin's Bacteriophage in the treatment of infections with the above mentioned bacteria.

### 3. Preparation of Pharmaceutical Compositions

For the preparation of a first pharmaceutical composition for use in the treatment of bacterial infections, a phage stock / phage lysate has been prepared according to the above described protocols. On the basis of the phage stock, a composition for intravenous application according to Table 4 has been prepared.

**Table 4: Content of a pharmaceutical composition according to the present invention**

| **component** | **concentration / amount** |
|---|---|
| bacteriophage | 10⁵ to 10⁷ PFU/ml |
| NaCl (sodium chloride) | 0.9 % by weight |
| glucose | 5.0 % by weight |
| amino acid solution | 10.0 % by volume |
| sterile water for injection purposes | ad. 100 % |

Additionally, a second pharmaceutical composition for use in the treatment of bacterial infections has been prepared on the basis of Caucasian Origin's Bacteriophage lysates generated from eight strains of host bacteria (*Staphylococcus aureus, Pseudomonas aeruginosa, Proteus vulgaris, Escherichia coli, Acinetobacter baumannii, Klebsiella pneumoniae, Enterobacter* spp. and *Staphylococcus pyogenes*). The host bacteria had been isolated from 50 patients suffering from bacterial sepsis (cf. lysis studies).

For the pharmaceutical composition, eight lysates of Caucasian Origin's Bacteriophage have been prepared according to the preparation protocols as described above. The host strains were *Staphylococcus aureus, Pseudomonas aeruginosa, Proteus vulgaris, Escherichia coli, Acinetobacter baumannii, Klebsiella pneumoniae, Enterobacter* spp. and *Staphylococcus pyogenes.* The eight lysates were combined. Bacterial cell parts and undesired residues were removed. A sterilization filtration was performed. The phage titer of the combined lysate composition was 1 x 10⁷ PFU/ml. Additionally, 0.01 % by weight, based on the composition, of quinosole as a preservative have been added. The composition has been used in a clinical trial on 50 patients suffering from acute bacterial sepsis.

### 4. Efficacy Studies

### Study design (first composition)

In two large studies (first and second efficacy study), the efficacy of the above described first pharmaceutical compositions as a pathogen-specific therapy for bacterial infections caused by staphylococci and several gram-negative bacteria *(Staphylococcus aureus, Staphylococcus aureus* methicillin-resistant, *Staphylococcus aureus* vancomycin-intermediate/resistant, *Acinetobacter baumannii* carbapenem-resistant, *Klebsiella pneumoniae, Escherichia coli, Enterobacter spp., Proteus mirabilis, Proteus vulgaris, Proteus faecium, Salmonella typhimurium, Salmonella enterica* and *Pseudomonas aeruginosa* carbapenem-resistant) has been evaluated.

In order to analyze the efficacy of the composition, 256 patients suffering from various bacterial infections with the aforementioned pathogenic bacteria have been consulted and treated with the above described composition. For calculation of the individual dosage, all patients were initially weighted. The bacteriophage treatment lasted up to 8 weeks, with an average duration of treatment of 7 to 28 days.

During bacteriophage treatment, the patients received intravenously a daily dose of the composition of 15 to 20 ml per kg bodyweight. In cases of seriously ill patients (according to the standards of the American Society of Health-System Pharmacists, the Infectious Diseases Society of America and the Society of Infectious Diseases Pharmacists), an initial loading dose of 25 ml per kg bodyweight was applied. During further treatment, a daily dose of 1.5 to 20 ml/kg was applied.

Furthermore, in order to determine the specificity of the bacteriophage, the infections have been analyzed with respect to their bacterial colonization. For this purpose, bacteria have been isolated from the infection. The bacteria have been further analyzed by using microbiological standard techniques, in particular RT-PCR, PCR, fluorescent spectrophotometers as well as microscopy (fluorescent microscope, inverted microscope, video microscope).

### First efficacy study (first composition)

As delineated above, 256 cases of suppurative bacterial infections caused by staphylococci and Gram-negative bacteria (*Acinetobacter baumannii* carbapenem-resistant, *Klebsiella, Escherichia, Proteus* and *Pseudomonas aeruginosa* carbapenem-resistant) have been treated with the pharmaceutical composition according to the present invention. Positive therapeutic effects were obtained in 168 cases, i.e. 65.625 %. It was found that bacteriophage therapy effectively controls the infections process irrespective of its localization, patient age and sex, and type of infection (mono-infections, poly-infections). The highest effectiveness of the bacteriophage therapy was noted in furunculosis (100 % of the treated patients cured). High effectiveness (over 90 % of the treated patients cured) was also observed in osteomyelitis, infections of connective tissue and lymphatic vessels, as well as chronic suppurative fistulas and in oncological patients with High CRP level. Overall, the use of the bacteriophage according to the present invention for therapy of bacterial infections revealed very positive results, where antibiotics were ineffective.

### Second efficacy study (first composition)

The results of the second efficacy study concerning the therapy of bacterial infections on the basis of the bacteriophage according to the present invention are described below. As can be seen, bacteriophage therapy was highly effective in the treatment of infections caused by different species of bacteria, as *Escherichia, Klebsiella, Proteus, Enterobacter, Pseudomonas* and *Staphylococcus aureus* (furunculosis).

One the one hand, the bacteria which had been causal for the infection were determined. Thereby, it was found that 2,738 strains (69.2 % of all isolated strains) were isolated from infections caused by one species of bacteria (mono-infections), the great majority by *Staphylococcus aureus* (1,674 strains). The remaining 1,218 strains (30.8 % of all isolated strains) were isolated from infections caused by several species of bacteria (poly-infections). *Staphylococcus* and *Pseudomonas* occurred more frequently in mono-infections, wherein *Klebsiella, Escherichia, Enterobacter* and *Proteus* occurred more frequently in poly-infections.

In 187 in vitro tests (85 %) treated with the bacteriophage according to the present invention, a complete recovery or healing of the local lesions was obtained (range 64 to 100 %), according to the type of infection. Noteworthy is that bacteriophage therapy was most effective in purulent meningitis, *Acinetobacter baumannii* (carbapenem-resistant), *Pseudomonas aeruginosa,* (carbapenem-resistant), *Enterobacteriaceae* (carbapenem-resistant), *Enterococcus faecium* (vancomycin-resistant), *Salmonella enterica* (fluoroquinolone-resistant), *Staphylococcus aureus* (methicillin-resistant, vancomycin-intermediate and resistant) and furunculosis (90 to 100 % cured). High effectiveness was also noted in septicemia of different origin, purulent otitis media, suppurative peritonitis, pyogenic arthritis and myositis, osteomyelitis of the long bones, suppurative osteitis after bone fractures, pyogenic infections of burns, purulent mastitis and chronic suppurative fistulas. In 22 cases (10 %) transient improvement was observed and in 11 cases only (5 %) bacteriophage therapy was found to be ineffective. Of particular importance is that two - due to the pathogenicity combined with multiple antibiotic resistances - dangerous pathogens, namely *Staphylococcus aureus* and *Pseudomonas aeruginosa,* which frequently cause serious infections, were highly sensitive to our specific phage (95 and 89 %, respectively).

### Clinical Trial (second composition)

Additionally, the second pharmaceutical composition was used in a clinical trial for the treatment of 50 patients suffering from acute moderate bacterial sepsis. In this trial, in 41 cases the treatment was successful, i.e. no supplementation of therapy with additional antibiotics was necessary. None of the patients suffered from undesired side effects. Overall, in the clinical trial 82 % of the treated sepsis cases were cured without using antibiotics. A treatment without antibiotics is not only advantageous with respect to reduced side effects but also with respect to a containment of the development resistances of antibiotics.

### 5. Summary

The results confirm the effectiveness of Caucasian origin's bacteriophage therapy in the combating of antibiotic-resistant bacterial infections. In fact, the results suggest that Caucasian Origin's Bacteriophage therapy is more effective than antibiotic treatment. It can be concluded that Caucasian Origin's Bacteriophage therapy is a highly efficient replacement of treatment with antibiotics, particularly with respect to infections with antibiotic resistant bacteria.

## Claims

1. A pharmaceutical composition for the use in the treatment of bacterial infections and/or infectious diseases, wherein the composition contains a therapeutically effective amount of a bacteriophage, wherein the bacteriophage is a Caucasian Origin's Bacteriophage as deposited at Deutsche Stammsammlung von Mikroorganismen und Zellkulturen GmbH (DSMZ) under the accession no. DSM 33404.

2. The pharmaceutical composition for the use in the treatment of bacterial infections and/or infectious diseases according to claim 1, wherein the composition has a phage titer in the range of 1 to 10¹⁵ PFU/ml, especially 10² to 10¹³ PFU/ml, in particular 10³ to 10¹¹ PFU/ml, preferably 10⁴ to 10⁹ PFU/ml, particularly preferred 10⁵ to 10⁷ PFU/ml, based on the composition.

3. The pharmaceutical composition for the use in the treatment of bacterial infections and/or infectious diseases according to claim 1 or 2, wherein composition comprises the Caucasian Origin's Bacteriophage in the form of a phage lysate, particularly wherein the lysate has been obtained from Caucasian Origin's Bacteriophage infected *Staphylococcus aureus, Pseudomonas aeruginosa, Proteus vulgaris, Escherichia coli, Acinetobacter baumannii, Klebsiella pneumoniae, Enterobacter* spp. and/or *Staphylococcus pyogenes.*

4. The pharmaceutical composition for the use in the treatment of bacterial infections and/or infectious diseases according to any of the preceding claims, wherein the composition is present as an aqueous composition and/or wherein the composition is aqueously based and/or is present as an aqueous formulation, especially in the form of an aqueous solution, and/or wherein the composition contains water, especially purified water, and/or wherein the composition contains water as pharmaceutically compatible excipient, and/or wherein the composition is present as an isotonic composition, especially solution, in relation to preferably mammalian, in particular human blood and/or blood cells.

5. The pharmaceutical composition for the use in the treatment of bacterial infections and/or infectious diseases according to any of the preceding claims, wherein the composition contains at least one physiologically safe or acceptable, osmotically active substance and/or at least one physiologically safe or acceptable osmolyte, in particular wherein the osmotically active substance and/or osmolyte is selected from the group of ions of physiologically safe or acceptable salts, preferably alkali ions, in particular sodium ions and chloride ions; sugars, in particular glucose; polyols, in particular glycerol; and/or amino acids.

6. The pharmaceutical composition for the use in the treatment of bacterial infections and/or infectious diseases according to any of the preceding claims, wherein the composition is prepared for an intravenous, subcutaneous, intramuscular, oral, parenteral and/or topical application, especially an intravenous, topical and/or oral application, preferably an intravenous and/or oral application.

7. The pharmaceutical composition for the use in the treatment of bacterial infections and/or infectious diseases according to any of the preceding claims, wherein the composition comprises at least one customary pharmaceutical additive and/or excipient, which is preferably selected from the group of processing aids, stabilizers, emulsifiers, antioxidants, preservatives, humectants, pH setters, pH buffer substances, thickeners, antiseptics, dyes, buffer substances, odorants, fragrances, extenders, binders, wetting substances and/or preservatives and the combinations thereof.

8. The pharmaceutical composition for the use in the treatment of bacterial infections and/or infectious diseases according to any of the preceding claims, wherein infections and/or diseases are caused by bacteria of the genera *Staphylococcus, Streptococcus, Enterobacter, Escherichia, Pseudomonas, Klebsiella, Acinetobacter, Proteus* and/or *Salmonella,* in particular by bacteria of the species *Klebsiella pneumoniae, Pseudomonas aeruginosa, Escherichia coli, Enterobacter spp., Acinetobacter baumannii, Proteus vulgaris, Staphylococcus aureus, Streptococcus pyogenes* and/or *Salmonella enterica.*

9. The pharmaceutical composition for the use in the treatment of bacterial infections and/or infectious diseases according to any of the preceding claims, wherein the infectious diseases are selected from furunculosis, osteomyelitis, infections of connective tissue, infections of lymphatic vessels, fistulas, septicemia, meningitis, otitis, peritonitis, arthritis, myositis, osteomyelitis, osteitis, infections of wounds and/or burns, infections of skin and/or mastitis.

10. Bacteriophage for the use in the treatment of bacterial infections and/or infectious diseases, wherein the bacteriophage is a Caucasian Origin's Bacteriophage as deposited at Deutsche Stammsammlung von Mikroorganismen und Zellkulturen GmbH (DSMZ) under the accession no. DSM 33404.

11. Bacteriophage according to claim 10 for the use in the treatment of bacterial infections and/or infectious diseases, wherein the infections and/or diseases are caused by bacteria of the genera *Staphylococcus, Streptococcus, Enterobacter, Escherichia, Pseudomonas, Klebsiella, Acinetobacter, Proteus* and/or *Salmonella,* in particular by bacteria of the species *Klebsiella pneumoniae, Pseudomonas aeruginosa, Escherichia coli, Enterobacter spp., Acinetobacter baumannii, Proteus vulgaris, Staphylococcus aureus, Streptococcus pyogenes* and/or *Salmonella enterica.*

12. Bacteriophage according to claim 10 or 11 for the use in the treatment of bacterial infections and/or infectious diseases, wherein the infectious diseases are selected from furunculosis, osteomyelitis, infections of connective tissue, infections of lymphatic vessels, fistulas, septicemia, meningitis, otitis, peritonitis, arthritis, myositis, osteomyelitis, osteitis, infections of wounds and/or burns, infections of skin and/or mastitis.


SEQUENCE LISTING

<110>  Phagegroup LLC

<120>  Bacteriophage Based Therapy

<130>  19.6176

<160>  4

<170>  PatentIn version 3.5

<210>  1
<211>  141
<212>  RNA
<213>  Caucasian Origins BP

<400>  1
augaaaaauu uuuccuauug ucuuauuguu ucgcccaccg cugcauacca ccaggaugag      60

uguuggcguc uacggaguag cagaggugga agaggagggc caauggcgca gaacauguuc     120

uuguauuguc aacggaggau g                                               141


<210>  2
<211>  192
<212>  RNA
<213>  Caucasian Origins BP

<400>  2
augggcggua ugauguuacc cacuaaagau uaucauccac ggaggggugc caacguguuu      60

ucacaauucu cacaccagaa caagcgccga agaaugucua auagugggau gaugaugaaa     120

auggguccca gcagacaaau ggcugccgca gugguuccuu accacaacaa aaagcaaaau     180

uucaacccgu ga                                                         192


<210>  3
<211>  128
<212>  PRT
<213>  Caucasian Origin's Bacteriophage


<220>
<221>  Capsid_Protein
<222>  (1)..(128)

<400>  3

Met Asn Pro Leu Ile Leu Lys Leu Leu Pro Val Gly Leu Val Tyr Ala
1               5                   10                  15


Asp Leu Lys Thr Thr Thr Phe Ser Val Lys Leu Ser Tyr Ser Pro Pro
            20                  25                  30


Thr Thr Tyr Ser Pro Ile Arg His Leu His Cys Pro Ile Trp Gly Thr
        35                  40                  45

Arg Thr Gly Ile His Ser Glu Lys Ile His Leu Ile Leu Phe Ala Thr
    50                  55                  60

Ala Thr Leu Trp Leu Leu Gly Thr Thr Thr Ser Asn Tyr Leu Val Leu
65                  70                  75                  80

Pro Tyr Tyr Thr Trp His Lys Arg Leu Leu Arg Pro Arg Gly Thr Val
                85                  90                  95

Arg Leu Trp Ile Asp Phe Ser His Asp Asn Asp Tyr Asn Gly His Gly
            100                 105                 110

Phe Ser Arg Asn Ala Ile Arg Thr His Phe Ser Asp Ala His Gly Ala
        115                 120                 125

<210>  4
<211>  384
<212>  RNA
<213>  Caucasian Origin's Bacteriophage

<220>
<221>  mRNA_CapsidProtein
<222>  (1)..(384)

<400>  4
augaauccuu uaauccuuaa acuucuaccc gugggauugg uuuaugccga uuuaaaaacg      60

acgacguuua guguaaagcu aucuuauagc ccucccacga cguauucccc cauccgucau     120

cuucauugcc caauuugggg gacgcggacg gggauccauu cugagaaaau ccaccuaauc     180

cuuuuugcua cggcaaccuu augguuguug gggacgacga cgagcaauua ucuuguccuc     240

cccuauuaua cauggcauaa acgccuacua cgcccucgcg guacggucag acucuggauc     300

gauuucucuc acgacaacga cuacaacggc cacggcuucu cccguaacgc uauacggacg     360

cacuuuucgg acgcccacgg cgcc                                            384